# EUROPÄISCHE PATENTSCHRIFT

(11) **EP 0 879 608 B1**
(45) Veröffentlichungstag und Bekanntmachung des Hinweises auf die Patenterteilung: **29.12.2004**
(21) Anmeldenummer: 98105083.4
(22) Anmeldetag: 20.03.1998
(51) Int. Cl.: A61M 1/02

(54) **Vorrichtung zum Trennen von Blut in Blutkomponenten**
Means for separating blood in its components
Dispositif de separation de sang en ses composants

(30) Priorität: 24.03.1997 DE 19712298
(43) Veröffentlichungstag der Anmeldung: 25.11.1998
(73) Patentinhaber: Fresenius AG, 61352 Bad Homburg v.d.H. (DE)
(72) Erfinder: Mari, Giorgio, 41037 Mirandola (IT); Verri, Paolo, 41031 Concordia (IT)
(74) Vertreter: Luderschmidt, Schüler & Partner GbR

(56) Entgegenhaltungen:
- EP-A- 0 714 667
- US-A- 4 997 577

## Beschreibung

Die Erfindung betrifft eine Vorrichtung zum Trennen von Blut in Blutkomponenten.

Die EP 0 349 188 B 1 beschreibt eine Vorrichtung zum Trennen von Blut in Blutkomponenten, die einen Blutsammelbeutel aufweist, der über eine erste Schlauchleitung mit einem Primärbeutel verbunden ist, wobei der Primärbeutel über eine weitere Schlauchleitung mit einem Satellitenbeutel verbunden ist. In der ersten ScbJauchleitung, die den Blutsammelbeutel mit dem Primärbeutel verbindet, ist ein Filter zur Entfernung von Leukozyten angeordnet. Zur Gewinnung von leukozytenfreien Blutkomponenten wird Vollblut eines Spenders in dem Blutsammelbeutel gesammelt und über die den Filter enthaltende Schlauchleitung in den Primärbeutel überführt. Daraufhin wird der zwischen dem Filter und dem Primärbeutel liegende Abschnitt der Schlauchleitung durchtrennt, wobei die Trennstelle abgedichtet wird. Der Primärbeutel wird dann zusammen mit dem Sekundärbeutel zentrifugiert, um das in dem Primärbeutel befindliche Blut in zwei Blutkomponenten aufzutrennen. Eine der beiden Blutkomponenten wird dann über eine zweite Schlauchleitung in den Satellitenbeutel überführt Auf diese Weise können nach der Filtration des Vollblutes leukozytenfreie Blutkomponenten gewonnen werden. Ein entscheidender Vorteil wird bei dem bekannten Verfahren darin gesehen, daß eine Zentrifugation der Beutelanordnung ohne den Filter erfolgt und eine mechanische Beanspruchung des Filters somit vermieden wird.

Aus der US-A-4,596,657 ist eine Vorrichtung zur Trennung von Blut in Blutkomponenten bekannt, die einen Primärbeutel aufweist, der über eine erste Schlauchleitung mit einem ersten Satellitenbeutel und über eine zweite Schlauchleitung mit einem zweiten Satellitenbeutel verbunden ist, der eine Zusatzlösung enthält. Der Leukozytenfilter ist bei der bekannten Vorrichtung in der zweiten Schlauchleitung angeordnet, die den Primärbeutel mit dem zweiten Satellitenbeutel verbindet. Zur Gewinnung eines leukozytenfreien Erythrozytenkonzentrats wird das in dem Primärbeutel gesammelte Vollblut des Spenders zunächst zentrifugiert, um das Vollblut in eine Mischschicht aus Blutplasma und Thrombozyten und eine Erythrozytenschicht aufzutrennen. Die Mischschicht aus Blutplasma und Thrombozyrten wird dann in den ersten Satellitenbeutel überführt. Daraufhin wird die Zusatzlösung aus dem zweiten Satellitenbeutel in den Primärbeutel überführt. Die mit der Zusatzlösung resuspendierten Erythrozyten werden dann aus dem Primärbeutel über die den Leukozytrenfilter enthaltende zweite Schlauchleitung in den zweiten Satellitenbeutel überführt. Die bekannte Vorrichtung ist nicht für eine Vollblutfiltration bestimmt.

Die US-A-5 100 564 beschreibt ein Beutelsystem, das einen Sammelbeutel und zwei Satellitenbeutel umfaßt, wobei die Satellitenbeutel jeweils über Leitungen mit dem Sammelbeutel verbunden sind. Diese Leitungen enthalten jeweils einen Filter. Das Vollblut wird in dem Sammelbeutel gesammelt und zur Trennung in seine Komponenten zentrifugiert. Erst dann erfolgt die Filtration der einzelnen Blutkomponenten. Das bekannte Beutelsystem ist nicht zur Vollblutfiltration bestimmt.

Die US-A-5 527 472 beschreibt ein Beutelsystem, das einen Sammelbeutel und verschiedene weitere Beutel umfaßt, die durch flexible Leitungen miteinander verbunden sind. In einer der Leitungen ist ein Filter enthalten. Der Entfernung von Restluft dient eine Bypassleitung. Das bekannte Beutelsystem ist nicht zur Vollblutfiltration bestimmt.

Die EP 0 714 667 A2 beschreibt eine Vorrichtung zum Trennen von Blut in Blutkomponenten, die zwei miteinander zu verbindende Behälteranordnungen umfasst. Die erste Behälteranordnung weist einen Primärbehälter auf, der über eine Schlauchleitung mit einem Satellitenbehälter verbunden ist. In dem Primärbehälter wird das Blut des Patienten gesammelt und durch Zentrifugieren in verschiedene Blutkomponenten getrennt. Die zweite Behälteranordnung weist eine Sammelleitung auf, in der ein Filter zur Entfernung bestimmter Blutkomponenten angeordnet ist. Zur Schaffung einer Fluidverbindung unter Umgehung des Filters ist eine By-Pass-Leitung vorgesehen.

Der Erfindung liegt die Aufgabe zugrunde, eine universell einsetzbare Vorrichtung zur Trennung von Blut in Blutkomponenten zu schaffen, die sowohl die Filtration nur einer Blutkomponente als auch eine Vollblutfiltration erlaubt.

Die Lösung der Aufgabe erfolgt erfindungsgemäß mit den Merkmalen des Patentanspruchs 1.

Bei der erfindungsgemäßen Vorrichtung zum Trennen von Blut in Blutkomponenten ist eine By-Pass-Leitung zur Schaffung einer Fluidverbindung unter Umgehung des Filters zwischen dem Sammelbehältnis und dem Primärbehältnis vorgesehen. Wenn nicht eine Vollblutfiltration, sondern die Filtration nur einer Blutkomponente erfolgen soll, wird das in dem Sammelbehältnis gesammelte Blut des Spenders über die By-Pass-Leitung unter Umgehung des Filters aus dem Sammelbehältnis in das Primärbehältnis überführt. Das in dem Primärbehältnis befindliche Blut wird dann durch Zentrifugieren in zwei Blutkomponenten aufgetrennt, von denen eine in ein dem Primärbehältnis nachgeschaltetes Satellitenbehältnis überführt wird. Die in dem Primärbehältnis befindliche Blutkomponente wird nun zum Trennen der Leukozyten über den das Leukozytenfilter enthaltenden Leitungsweg in den Sammelbehälter überführt.

Die Mittel zum Öffnen und Schließen der By-Pass-Leitung bzw. der Sammelleitung sind vorzugsweise Schlauchklemmen, die an den Leitungen befestigt sind. Die Schlauchklemmen können aber auch lose dem Schlauchleitungssystem beigelegt sein.

Ein leukozytenfreies Erythrozytenkonzentrat wird dadurch gewonnen, daß das Vollblut in dem Primärbehältnis durch Zentrifugieren in eine Erythrozytenschicht und eine Schicht aus Blutplasma und Thrombozyten aufgetrennt und die Mischschicht aus Blutplasma und Thrombozyten in das Satellitenbehältnis überführt wird, wobei die Erythrozyten in dem Primärbehältnis verbleiben. Die Erythrozyten werden dann zur Gewinnung des leukozytenfreien Erythrozytenkonzentrats über den das Leukozytenfilter enthaltenden Leitungsweg in das Sammelbehältnis überführt. Das Sammelbehältnis kann dann abgetrennt und die Trennstelle abgedichtet werden.
Alternativ ist mit der erfindungsgemäßen Vorrichtung auch eine Trennung der Blutkomponenten nach dem Verfahren möglich, das in der EP 0 349 188 beschrieben ist. Hierzu wird die By-Pass-Leitung unterbrochen und das Vollblut wird zuerst über den das Leukozytenfilter enthaltende Leitungszweig dem Primärbehältnis zugeführt, bevor es durch Zentrifugieren in die einzelnen Blutkomponenten aufgetrennt wird. Dabei ist der Leukozytenfilter derart ausgebildet, daß er in zwei Richtungen betrieben werden kann, d.h. die Filteranschlüsse sowohl als Einlaß als auch als Auslaß verwendet werden können.

Zur Gewinnung eines leukozytenfreien Erythrozytenkonzentrats aus Vollblut reicht eine Trennvorrichtung mit nur einem Satellitenbehältnis aus. Eine vorteilhafte Ausführungsform der Trennvorrichtung umfaßt ein zweites Satellitenbehältnis, das dem ersten Satellitenbehältnis nachgeschaltet ist, so daß in einem zweiten Zentrifugationsschritt die in dem ersten Satellitenbehältnis befindlichen Blutkomponenten aufgetrennt und die abgetrennte Blutkomponente in das zweite Satellitenbehältnis überführt werden kann.

Bei einer bevorzugten Ausführungsform der Trennvorrichtung ist ein Zusatzmittelbehältnis vorgesehen, das über eine Zusatzmittelleitung mit dem Primärbehältnis verbunden ist. Das Zusatzmittelbehältnis ist mit einer Lösung zur Lagerung einer Blutkomponente befüllt. Diese Lösung kann der in dem Primärbehältnis verbleibenden Blutkomponente zugeführt werden.

In bevorzugter Ausgestaltung ist die erste Sammelleitung, die das Sammelbehältnis mit dem Filtereinfaß verbindet, und/oder die zweite Sammelleitung, die den Filterauslaß mit dem Primärbehältnis verbindet, als durchtrennbare oder abdichtbare oder abklemmbare Schlauchleitung, z.B. eine PVC-Schlauchleitung ausgebildet, so daß sich das Sammelbehältnis zur Lagerung der leukozytenfreien Blutkomponenten leicht separieren läßt bzw. sich der Leukozytenfilter von dem Primärbehältnis vor dem Zentrifugieren abtrennen läßt. Zum Unterbrechen der Fluidverbindung können an den Schlauchleitungen herkömmliche Schlauchklemmen vorgesehen sein. Die Schlauchleitungen können aber auch mit den bekannten Verfahren nach dem Abtrennen verschweißt werden.

Im folgenden werden zwei Ausführungsbeispiele der Erfindung unter Bezugnahme auf die Zeichnungen näher erläutert.

Es zeigen:
- Figur 1: eine erste Ausführungsform einer Vorrichtung zur Trennung von Blut in Blutkomponenten mit einem Satellitenbehältnis in schematischer Darstellung,
- Figur 2: einen Schnitt durch den Filter zur Entfernung von Leukozyten und
- Figur 3: eine zweite Ausführungsform der Trennvorrichtung mit zwei Satellitenbehältnissen.

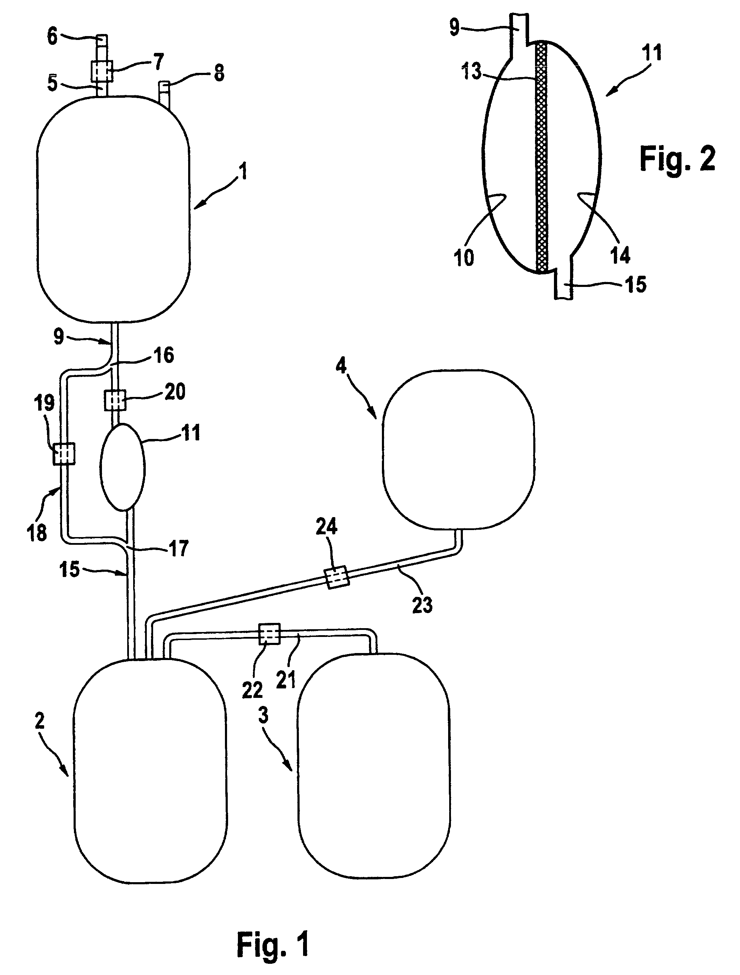

Eine Ausführungsform einer Vorrichtung zur Trennung von Blut in Blutkomponenten umfaßt ein Sammelbehältnis 1, ein Primärbehältnis 2, ein Satellitenbehältnis 3 und ein Zusatzmittelbehältnis 4, die als Folienbeutel ausgebildet sind (Figur 1). Der ein Antigerinnungsmittel enthaltende Sammelbeutel 1 weist eine Anschlußleitung 5 mit einem Luer-Lock-Konnektor 6 zum Anschluß einer nicht dargestellten Blutzuführleitung auf, die mit einer Kanüle versehen ist. Die Blutzuführleitung mit der Kanüle kann aber auch Bestandteil der Trennvorrichtung sein. Zum Abklemmen der Anschlußleitung 5 ist an dieser eine Schlauchklemme 7 vorgesehen. Darüber hinaus verfügt der Sammelbeutel 1 über einen Spike-Konnektor 8 zum Anschluß einer nicht dargestellten Schlauchleitung zum späteren Überführen der in dem Sammelbeutel gesammelten Blutkomponente zu dem Patienten.

Der Sammelbeutel 1 ist über eine erste Sammelleitung 9 mit der ersten Kammer 10 eines Filters 11 zur Entfernung von Leukozyten verbunden, der durch ein Filtermaterial 13 in die erste Kammer 10 und eine zweite Kammer 14 unterteilt ist (Figur 2). Die zweite Kammer 14 des Leukozytenfilters 11 ist über eine zweite Sammelleitung 15 mit dem Primärbeutel 2 verbunden. Die erste und zweite Sammelleitung 9, 15 sind PVC-Leitungen, die durchtrennt und mit den bekannten Schweißvorrichtungen flüssigkeitsdicht abgedichtet werden können.

Zwischen dem Sammelbeutel 1 und dem Leukozytenfilter 11 weist die erste Sammelleitung 9 eine erste Abzweigung 16 auf, während die zweite Sammelleitung 15 zwischen dem Filter 11 und dem Primärbeutel 2 eine zweite Abzweigung 17 aufweist. Zur Umgehung des über den Leukozytenfilter 11 führenden Leitungsweges 9, 15 ist eine By-Pass-Leitung 18 vorgesehen, deren Enden an der ersten bzw. zweiten Abzweigung 16 bzw. 17 angeschlossen sind.

An der By-Pass-Leitung 18 und an dem zwischen der ersten Abzweigung 16 und dem Leukozytenfilter 11 liegenden Abschnitt der ersten Sammelleitung 9 sind jeweils eine Schlauchklemme 19 bzw. 20 vorgesehen. Anstelle der Schlauchklemmen 19, 20 in der By-Pass-Leitung 18 bzw. der ersten Sammelleitung 9 können aber auch Zwei-Wege-Ventile in der ersten bzw. zweiten Sammelleitung 9, 15 vorgesehen sein.

Der Primärbeutel 2 ist über eine erste Überführungsleitung 21, die mit einer Schlauchklemme 22 versehen ist, mit dem ersten Satellitenbeutel 3 verbunden. Der mit einer Zusatzlösung zur Lagerung von Erythrozyten befüllte Zusatzmittelbeutel 4 ist über eine Zusatzmittelleitung 23, die mit einer Schlauchklemme 24 versehen ist, mit dem Primärbeutel 2 verbunden. Die Zusatzlösung ist z.B. eine Sudiumchlorid (140mmol/l), Adenine (1,25mmol/l), Glukose (46mmol/l) und Mannitol (29mmol/l) enthaltende Lösung (SAGM-Lösung)

Das Verfahren zur Gewinnung von leukozytenfreiem Erythrozytenkonzentrat aus Vollblut wird nachfolgend im einzelnen beschrieben.

Nachdem alle Schlauchklemmen geschlossen sind, wird eine Blutzuführleitung mit einer Kanüle zur Entnahme des Spenderblutes an den Luer-Lock-Konnektor 6 der Anschlußleitung 5 angeschlossen und die Schlauchklemme 7 der Anschlußleitung wird geöffnet. Nach dem Befüllen des Sammelbeutels 1 mit dem Blut des Spenders wird die Schlauchklemme 7 wieder geschlossen. Dann wird die Schlauchklemme 19 der By-Pass-Leitung 18 geöffnet, so daß das Blut aus dem Sammelbeutel 1 unter Einfluß der Schwerkraft in den Primärbeutel 2 fließt. Daraufhin wird die Schlauchklemme 19 der By-Pass-Leitung wieder geschlossen und das in dem Primärbeutel 2 befindlichen Blut wird in bekannter Weise durch Zentrifugieren in eine Erythrozytenschicht und eine Mischschicht aus Blutplasma und Thrombozyten (PRP) aufgetrennt. Die oben liegende Mischschicht aus Blutplasma und Thrombozyten wird nun nach Öffnen der Schlauchklemme 22 der Überführungsleitung 21 in den Satellitenbeutel 3 überführt. Anschließend wird die Schlauchklemme 22 der Überführungsleitung 21 wieder geschlossen und die Schlauchklemme 24 der Zusatzmittelleitung 23 wird geöffnet, um die Zusatzlösung unter Einfluß der Schwerkraft in den Primärbeutel 2 zu überführen. Die mit der Zusatzlösung resuspendierten Erythrozyten werden dann nach dem Öffnen der Schlauchklemme 20 der ersten Sammelleitung 9 über den Leitungsweg, der den Leukozytenfilter 11 enthält, wieder in den Sammelbeutel 1 überführt, woraufhin die Schlauchklemme 20 wieder geschlossen wird. Anschließend wird die erste Sammelleitung 9 oberhalb der ersten Abzeigung 16 durchtrennt und die Trennstelle wird abgedichtet, so daß der das leukozytenfreie Erythrozytenkonzentrat enthaltende Sammelbeutel 1 entnommen werden kann.

Wenn die Schlauchklemme 19 der By-Pass-Leitung 18 geschlossen und die Schlauchklemme der ersten Sammelleitung 9 geöffnet ist, kann alternativ das zunächst in dem Sammelbeutel 1 gesammelte Vollblut nach dem eingangs beschriebenen Verfahren zur Entfernung der Leukozyten filtriert werden, um erst dann die einzelnen Blutkomponenten abzutrennen. Diesbezügl. wird nochmals ausdrücklich auf die EP 0 349 188 B1 verwiesen.

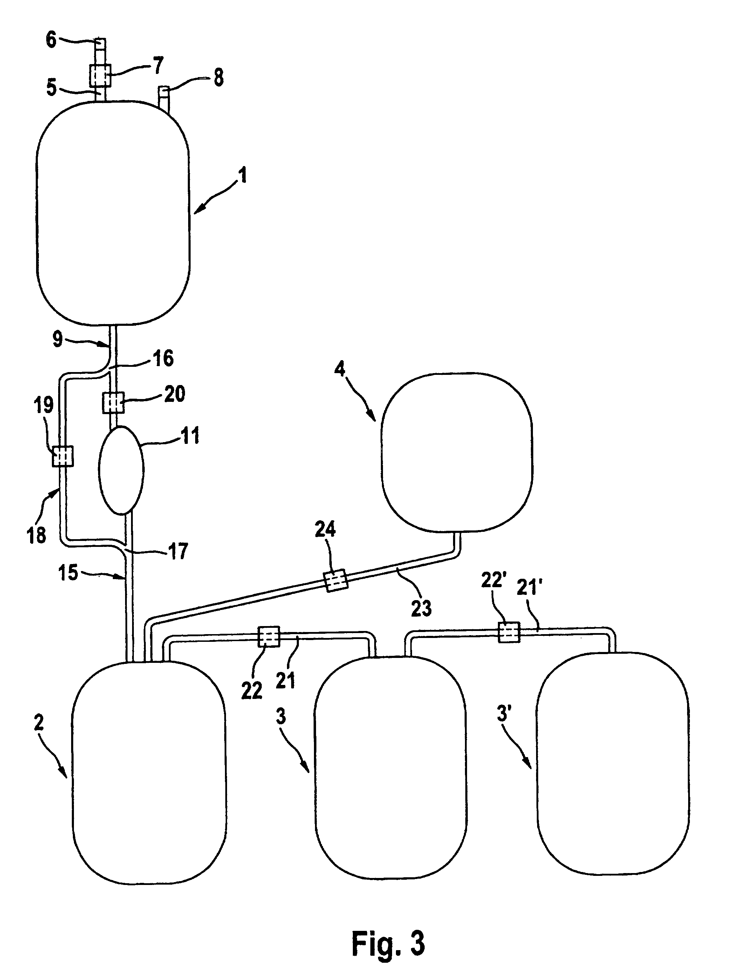

Figur 3 zeigt eine zweite Ausführungsform der Trennvorrichtung. Diese Ausführungsform unterscheidet sich von der unter Bezugnahme auf die Figuren 1 und 2 beschriebenen Ausführungsformen durch den zweiten Satellitenbehälter 3', der über eine zweite Überführungsleitung 21', die eine weitere Schlauchklemme 22' enthält, mit dem ersten Satellitenbehältnis 2 verbunden ist. In Figur 3 sind die Teile, die den Teilen der Trennvorrichtung gemäß den Figuren 1 und 2 entsprechen, mit den gleichen Bezugszeichen versehen. Das zweite Satellitenbehältnis 3' erlaubt eine weitere Auftrennung der in dem ersten Satellitenbehältnis 3 befindlichen Mischschicht aus Blutplasma und Thrombozyten in einem weiteren Zentrifugationsschritt.

## Patentansprüche

1. Vorrichtung zum Trennen von Blut in Blutkomponenten mit
einem Sammelbehältnis (1) zum Sammeln von Blutplasma, Erythrozyten, Leukozyten und Thrombozyten enthaltendes Vollblut von einem Spender, das einen Anschluss (6) zum Zuführen des Vollblutes und einen Anschluss (8) zur Entnahme mindestens einer Blutkomponente aufweist,
einem durch ein Filtermaterial (13) in zwei Kammern (10, 14) unterteilten Filter (11) zur Entfernung von Leukozyten,
einem in eine Zentrifuge einzulegenden Primärbehältnis (2) zum Auftrennen des Vollblutes in mindestens zwei Blutkomponenten durch Zentifugieren,
einer ersten Sammelleitung (9), die das Sammelbehältnis (1) mit der ersten Kammer (10) des Filters (11) verbindet, und einer zweiten Sammelleitung (15), die die zweite Kammer (14) des Filters mit dem Primärbehältnis (2) verbindet,
einer Satellitenbehältnisanordnung, die eine oder mehrere Satellitenbehältnisse (3, 3') zur Aufnahme der mindestens einen durch Zentrifugieren zu gewinnenden Blutkomponenten umfasst und
einer Leitungsanordnung (21, 21'), die mindestens eine direkt von dem Primärbehältnis (2) abgehende und direkt zu dem mindestens einen Satellitenbehältnis (3, 3') führende Überführungsleitung (21,21') zum Verbinden des Satellitenbehältnisses (3) bzw. der Satellitenbehältnisse (3, 3') mit dem Primärbehältnis aufweist,
**dadurch gekennzeichnet,**
**dass** eine By-Pass-Leitung (18) zur Schaffung einer Fluidverbindung unter Umgehung des Filters (11) zwischen dem Sammelbehältnis (1) und dem Primärbehältnis (2) vorgesehen ist, und
**dass** Mittel (19, 20) zum Öffnen bzw. Verschließen der By-Pass-Leitung (18) und zum Öffnen bzw. Verschließen der ersten Sammelleitung (9) vorgesehen sind.

2. Vorrichtung nach Anspruch 1, **dadurch gekennzeichnet, dass** die Satellitenbehältnisanordnung ein erstes Satellitenbehältnis (3) und die Leitungsanordnung eine erste Überführungsleitung (21) umfaßt, die das erste Satellitenbehältnis (3) mit dem Primärbehältnis (2) verbindet.

3. Vorrichtung nach Anspruch 2, **dadurch gekennzeichnet, dass** die Satellitenbehältnisanordnung ein zweites Satellitenbehältnis (3') und die Leitungsanordnung eine zweite Überführungsleitung (21') umfasst, die das zweite Satellitenbehältnis (3') mit dem ersten Satellitenbehältnis (3) verbindet.

4. Vorrichtung nach einem der Ansprüche 1 bis 3, **dadurch gekennzeichnet, dass** ein Zusatzmittelbehältnis vorgesehen ist, das über eine Zusatzmittelleitung (23) mit dem Primärbehältnis (2) verbunden ist.

5. Vorrichtung nach Anspruch 4, **dadurch gekennzeichnet, dass** das Zusatzmittelbehältnis (4) mit einer Lösung zur Lagerung einer Blutkomponente befüllt ist.

6. Vorrichtung nach einem der Ansprüche 1 bis 5, **dadurch gekennzeichnet, dass** die erste Sammelleitung (9) als durchtrennbare und abdichtbare oder abklemmbare Schlauchleitung ausgebildet ist.

7. Vorrichtung nach einem der Ansprüche 1 bis 6, **dadurch gekennzeichnet, dass** die zweite Sammelleitung (15) als durchtrennbare und abdichtbare oder abklemmbare Schlauchleitung ausgebildet ist.

8. Vorrichtung nach einem der Ansprüche 1 bis 7, **dadurch gekennzeichnet, dass** die erste Sammelleitung (9) eine erste Abzweigung (16) und die zweite Sammelleitung (15) eine zweite Abzweigung (17) aufweisen, wobei die By-Pass-Leitung (18) die erste Abzweigung mit der zweiten Abzweigung verbindet.

## Claims

1. A device for the separation of blood into blood components with
a collecting container (1) for the collection from a donor of full blood containing blood plasma, erythrocytes, leucocytes and thrombocytes, which has a connection (6) for supplying the full blood and a connection (8) for removing at least one blood component,
a filter (11) divided by a filter material (13) into two chambers (10, 14), said filter being for the removal of leucocytes,
a primary container (2) to be placed into a centrifuge, said primary container being for the separation of the full blood into at least two blood components by centrifugation,
a first collecting line (9), which connects the collecting container (1) to the first chamber (10) of the filter (11), and a second collecting line (15), which connects the second chamber (14) of the filter to the primary container (2),
a satellite container arrangement, which includes one or more satellite containers (3, 3') for accommodating the at least one blood component to be obtained by centrifugation and
a line arrangement (21, 21') which has at least one transfer line (21, 21') for connecting the satellite container (3) or the satellite containers (3, 3') to the primary container, said transfer line departing directly from the primary container (2) and leading directly to the at least one satellite container (3, 3'),
**characterised in that**
a bypass line (18) is provided for creating a fluid connection bypassing the filter (11) between the collecting container (1) and the primary container (2), and
that means (19, 20) are provided for opening and closing the bypass line (18) and for opening and closing the first collecting line (9).

2. The device according to claim 1, **characterised in that** the satellite container arrangement includes a first satellite container (3) and the line arrangement a first transfer line (21), which connects the first satellite container (3) to the primary container (2).

3. The device according to claim 2, **characterised in that** the satellite container arrangement includes a second satellite container (3') and the line arrangement a second transfer line (21'), which connects the second satellite container (3') to the first satellite container (3).

4. The device according to any one of claims 1 to 3, **characterised in that** an addition-agent container is provided, which is connected via an addition-agent line (23) to the primary container (2).

5. The device according to claim 4, **characterised in that** the addition-agent container (4) is filled with a solution for the storage of a blood component.

6. The device according to any one of claims 1 to 5, **characterised in that** the first collecting line (9) is designed as a flexible-tube line capable of being split and sealed or clamped.

7. The device according to any one of claims 1 to 6, **characterised in that** the second collecting line (15) is designed as a flexible-tube line capable of being split and sealed or clamped.

8. The device according to any one of claims 1 to 7, **characterised in that** the first collecting line (9) has a first branch (16) and the second collecting line (15) has a second branch (17), whereby the bypass line (18) connects the first branch to the second branch.

## Revendications

1. Dispositif pour séparer le sang en composants sanguins, comprenant :
un récipient de collecte (1) pour recueillir le sang entier, contenant le plasma sanguin, des érythrocytes, des leucocytes et des thrombocytes, provenant d'un donneur, le dispositif comprenant un raccord (6) pour l'admission du sang entier, et un raccord (8) pour le prélèvement d'au moins un composant sanguin,
un filtre (11) subdivisé en deux chambres (10, 14) par un matériau filtrant (13) et destiné à supprimer les leucocytes ;
un récipient primaire (2) destiné à être mis en place dans une centrifugeuse pour séparer le sang entier en au moins deux composants sanguins par centrifugation,
une première conduite de collecte (9) qui relie le récipient de collecte (1) à la première chambre (10) du filtre (11), et une seconde conduite de collecte (15) qui relie la seconde chambre (14) du filtre au récipient primaire (2),
un système à récipient satellite, qui comprend un ou plusieurs récipients satellites (3, 3') pour recevoir ledit au moins un composant sanguin qu'il s'agit de récupérer par centrifugation, et
un système à conduite (21, 21') qui comprend au moins une conduite de transfert (21, 21') qui part directement du récipient primaire (2) et qui mène directement audit au moins un récipient satellite (3, 3') pour relier le récipient satellite (3) ou les récipients satellites (3, 3') au récipient primaire,
**caractérisé en ce que** :
- il est prévu une conduite de by-pass (18) pour réaliser une liaison fluidique en contournant le filtre (11) entre le récipient de collecte (1) et le récipient primaire (2), et
- il est prévu des moyens (19, 20) pour ouvrir ou fermer la conduite de by-pass (18) et pour ouvrir ou fermer la première conduite de collecte (9).

2. Dispositif selon la revendication 1, **caractérisé en ce que** le système à récipient satellite comprend un premier récipient satellite (3), et le système à conduite comprend une première conduite de transfert (21) qui relie le premier récipient satellite (3) au récipient primaire (2).

3. Dispositif selon la revendication 2, **caractérisé en ce que** le système à récipient satellite comprend un second récipient satellite (3'), et le système à conduite comprend une seconde conduite de transfert (21'), qui relie le second récipient satellite (3) au premier récipient satellite (3).

4. Dispositif selon l'une des revendications 1 à 3, **caractérisé en ce qu'**il est prévu un récipient pour produit additif, qui est relié au récipient primaire (2) via une conduite pour produit additif (23).

5. Dispositif selon la revendication 4, **caractérisé en ce que** le récipient pour produit additif (4) est rempli d'une solution pour le stockage d'un composant sanguin.

6. Dispositif selon l'une des revendications 1 à 5, **caractérisé en ce que** la première conduite de collecte (9) est réalisée sous forme d'une conduite en tuyau souple susceptible d'être coupée et d'être étanchée ou d'être pincée.

7. Dispositif selon l'une des revendications 1 à 6, **caractérisé en ce que** la seconde conduite de collecte (15) est réalisée sous forme d'une conduite en tuyau souple susceptible d'être coupée et d'être étanchée ou d'être pincée.

8. Dispositif selon l'une des revendications 1 à 7, **caractérisé en ce que** la première conduite de collecte (9) comporte une première dérivation (16) et la seconde conduite de collecte (15) comporte une seconde dérivation (17), la conduite de by-pass (18) reliant la première dérivation à la seconde dérivation.
